(19) Europäisches Patentamt
European Patent Office
Office européen des brevets

(11) Veröffentlichungsnummer: **0 208 265**
**B1**

(12) # EUROPÄISCHE PATENTSCHRIFT

(45) Veröffentlichungstag der Patentschrift: **23.08.89**

(21) Anmeldenummer: **86109074.4**

(22) Anmeldetag: **03.07.86**

(51) Int. Cl.⁴: **C 07 D 498/10,** C 07 D 519/00, C 08 K 5/35, C 09 D 7/12, C 09 K 15/20 // (C07D498/10, 263:00, 221:00)

(54) **Neue 1-Oxa-3,8-diaza-4-oxo-spiro- 4,5 -decan-Verbindungen und Verfahren zu ihrer Herstellung.**

(30) Priorität: **10.07.85 DE 3524541**

(43) Veröffentlichungstag der Anmeldung: **14.01.87 Patentblatt 87/3**

(45) Bekanntmachung des Hinweises auf die Patenterteilung: **23.08.89 Patentblatt 89/34**

(84) Benannte Vertragsstaaten: **CH DE GB LI**

(56) Entgegenhaltungen:
**EP-A-0 079 850**
**EP-A-0 095 076**
**DE-A-2 933 732**
**DE-A-3 149 453**
**DE-A-3 408 949**
**DE-A-3 523 679**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Ertl, Josef, Dr., Eichenstrasse 14, D-8857 Wertingen (DE)**

**EP 0 208 265 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft neue 1-Oxa-3,8-diaza-4-oxo-spiro-[4,5]-decan-Verbindungen, ihre Herstellung und Verwendung als Lichtschutzmittel für Polymere oder als Zwischenprodukte für die Herstellung von Kunststofadditiven.

Verbindungen der Formel

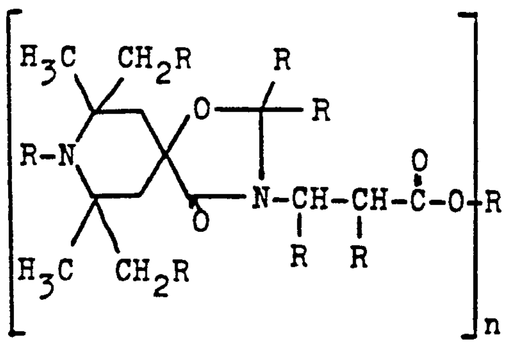

sind bekannt (vgl. Deutsche Offenlegungsschrift 3 149 453). Allerdings ist das Verfahren zu ihrer Herstellung kompliziert, da während der Reaktion das Reaktionsmedium mehrfach gewechselt wird, was zusätzliche Extraktionen und Destillationen bedingt. Außerdem besitzen die so hergestellten Verbindungen eine relativ hohe Flüchtigkeit.

Es wurde nun gefunden, daß wenig flüchtige Spirodecan-Verbindungen in einem einfachen Verfahren gewonnen werden können.

Gegenstand der Erfindung sind somit 1-Oxa-3,8-diaza-4-oxo-spiro-[4,5]-decan-Verbindungen der Formel I

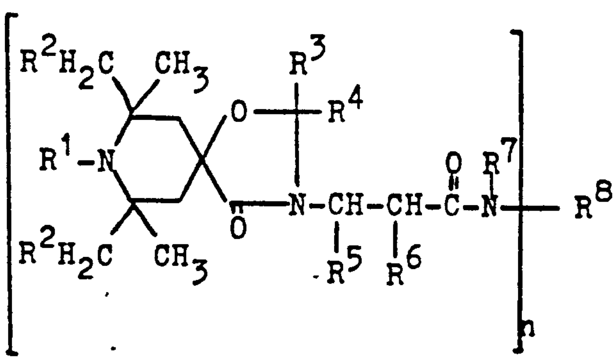

worin

n 1 oder 2 ist,

$R^1$ Wasserstoff, $C_1$-$C_4$-Alkyl, Benzyl, Allyl, $C_2$-$C_{30}$-Alkanoyl, $C_3$-$C_{20}$-Alkenoyl, $C_7$-$C_{11}$-Aroyl, $C_8$-$C_{14}$-Arylalkanoyl oder $C_8$-$C_{20}$-Alkylaryl ist,

$R^2$ Wasserstoff oder $C_1$-$C_4$-Alkyl bedeutet,

$R^3$ Wasserstoff, $C_1$-$C_{18}$-Alkyl, $C_5$-$C_{12}$-Cycloalkyl, eine Phenyl- oder Naphthylgruppe, die durch Chlor oder $C_1$-$C_4$-Alkyl substituiert sein kann, oder eine gegebenenfalls durch $C_1$-$C_4$-Alkyl substituierte $C_7$-$C_{12}$-Phenylalkylgruppe ist,

$R^4$ Wasserstoff, $C_1$-$C_4$-Alkyl, $C_5$-$C_{12}$-Cycloalkyl, durch -COOH, Carb-$C_1$-$C_4$-alkoxi oder Carbamoyl substituiertes $C_1$-$C_3$-Alkenyl, eine Phenyl-, Naphthyl- oder Pyridylgruppe, die durch $C_1$-$C_4$-Alkoxi oder $C_1$-$C_4$-Alkyl substituiert sein kann, oder eine $C_7$-$C_{12}$-Phenylalkylgruppe, die durch $C_1$-$C_4$-Alkyl substituiert sein kann, bedeutet, oder

$R^3$ und $R^4$ zusammen mit dem sie bindendem Kohlenstoffatom eine $C_5$-$C_{12}$-Cycloalkylgruppe, die durch eine bis vier $C_1$-$C_4$-Alkylgruppen substituiert sein kann, oder einen Rest der Formel

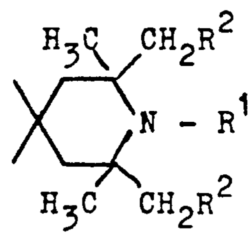

(II),

worin $R^1$ und $R^2$ die obengenannte Bedeutung haben, bilden,

$R^5$ Wasserstoff, Methyl, Phenyl oder Carb-$C_1$-$C_{21}$-alkoxi ist,

$R^6$ Wasserstoff oder Methyl bedeutet,

$R^7$ Wasserstoff, $C_1$-$C_{10}$-Alkyl oder einen Rest der Formel III

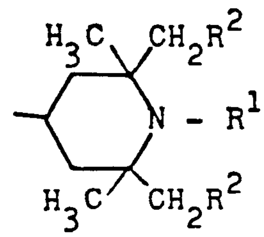

(III),

worin $R^1$ und $R^2$ die oben angegebene Bedeutung haben, bedeutet,

$R^8$ bei n = 1

Wasserstoff, $C_1$-$C_{21}$-Alkyl, $C_2$-$C_{22}$-Alkenyl, $C_7$-$C_{18}$-Phenylalkyl, $C_5$-$C_{12}$-Cycloalkyl, Phenyl, Naphthyl, $C_7$-$C_{18}$-Alkylphenyl, $C_2$-$C_{20}$-Alkyl, welches unterbrochen ist durch -O- oder

-N-
|
$R^9$

und/oder substituiert sein kann durch einen Rest der Formel IV

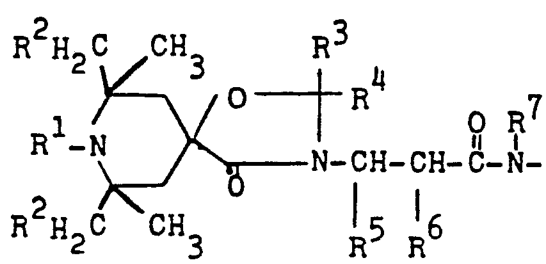

(IV).,

oder durch $C_1$-$C_{21}$-Alkylcarboxyl, wobei $R^1$, $R^2$, $R^3$, $R^4$ $R^5$, $R^6$ und $R^7$ die obige Bedeutung haben und $R^9$ Wasserstoff, $C_1$-$C_{10}$-Alkyl oder ein Rest der Formel III ist, bedeutet,

$R^8$ bei n = 2

geradkettiges oder verzweigtes $C_1$-$C_{30}$-Alkylen, $C_6$-$C_{15}$-Cycloalkylen, $C_2$-$C_{30}$-Alkenylen, Phenyldialkylen, wobei diese Reste durch -O- oder

-N-,
|
$R^9$

worin $R^9$ die obige Bedeutung hat, unterbrochen sein können, bedeutet.

$R^1$ ist bevorzugt Wasserstoff, $C_1$-$C_4$-Alkyl, $C_2$-$C_{18}$-Alkanoyl, beispielsweise Methyl, Ethyl, Propyl, Butyl, Acetyl, Propionyl, Butyryl, Lauroyl, Stearoyl, besonders bevorzugt Wasserstoff oder einer der genannten Säurereste. Insbesondere ist $R^1$ Wasserstoff.

$R^2$ ist bevorzugt Wasserstoff oder $C_1$-$C_4$-Alkyl, beispielsweise Methyl, Ethyl, Propyl, Butyl. Insbesondere ist $R^2$ Wasserstoff.

$R^3$ und $R^4$

sind unabhängig voneinander $C_1$-$C_{18}$-Alkyl, $C_5$-$C_{12}$-Cycloalkyl oder Phenyl, beispielsweise Ethyl, Butyl, Octyl, Lauryl, Stearyl, Cyclohexyl, Cyclodecyl, besonders bevorzugt $C_1$-$C_7$-Alkyl. Ins-

besondere sind $R^3$ und $R^4$ $C_1$-$C_4$-Alkyl, beispielsweise Methyl.

- $R^3$ und $R^4$ zusammen mit dem sie bindenden Kohlenstoffatom sind bevorzugt $C_5$-$C_{12}$-Cycloalkylen, besonders bevorzugt $C_6$- bzw. $C_{12}$-Cycloalkylen, insbesondere Cyclododecylen.
- $R^5$ ist bevorzugt Wasserstoff, Methyl oder Phenyl, besonders bevorzugt Wasserstoff.
- $R^6$ ist bevorzugt Wasserstoff oder Methyl. Insbesondere ist $R^6$ Wasserstoff.
- $R^7$ ist bevorzugt Wasserstoff oder 2,2,4,4-Tetramethylpiperid-4-yl.
- $R^8$ ist bevorzugt $C_1$-$C_{21}$-Alkyl, geradkettiges oder verzweigtes $C_1$-$C_{30}$-Alkylen, beispieleweise Methyl, Ethyl, Butyl, Ethylen, Butylen, Hexylen, besonders bevorzugt $C_2$-$C_{10}$-Alkylen. Insbesondere ist $R^8$ $C_2$-$C_6$-Alkylen beispielsweise Hexylen.

Die erfindungsgemäßen Verbindungen der Formel I werden hergestellt durch Umsetzung von Verbindungen der Formel V

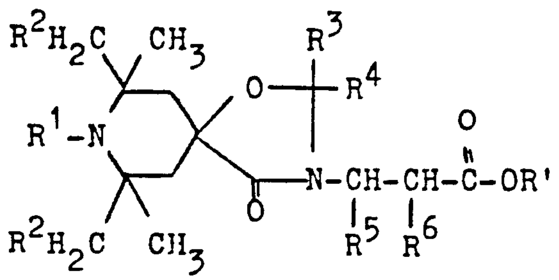

(V)

mit Aminen der Formel VI

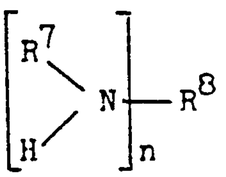

(VI)

In diesen Formeln
haben n, $R^1$, $R^2$, $R^3$, $R^4$, $R^5$, $R^7$ und $R^8$ die bereits angegebene Bedeutung.
R' ist $C_1$-$C_4$-Alkyl, vorzugsweise Methyl oder Ethyl.

Die Verbindungen der Formel V können gewonnen werden durch Umsetzung von Verbindungen der Formel VII

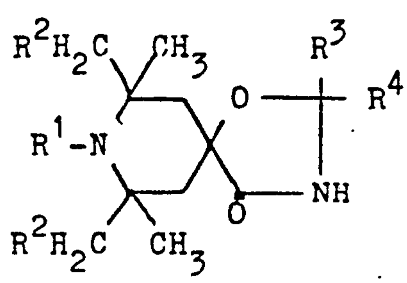

(VII),

mit Verbindungen der Formel VIII

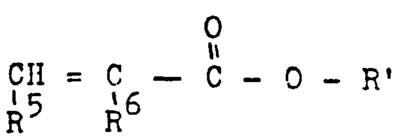

(VIII),

wobei $R^1$, $R^2$, $R^3$, $R^4$, $R^5$, $R^6$ und R' die obengenannte Bedeutung haben, bei einer Temperatur von 30 bis 150°C in Gegenwart eines basischen Katalysators und in Gegenwart von 0,05 bis 20 Mol-%, bezogen auf die Verbindung VII, eines Phasentransferkatalysators in einem bei Raumtemperatur flüssigen aromatischen Kohlenwasserstoff, vorzugsweise Toluol oder Xylol.

Als Phasentransferkatalysator wird vorzugsweise zugesetzt ein Polyethylenglykoldialkylether, ein substituiertes Phosphoniumsalz, beispieleweise ein Tetraalkylphosphoniumhalogenid, oder ein substituiertes

Ammoniumsalz, beispieleweise Tetraalkylammoniumhalogenid oder Trialkylbenzylammoniumhalogenid. Insbesondere wird Triethylbenzylammoniumchlorid oder Tetraalkylphosphoniumbromid zugesetzt. Die Menge beträgt 0,05 bis 20, vorzugeweise 0,1 bis 10, insbesondere 1 bis 10 Mol-%, bezogen auf die Verbindung der Formel VII.

Die Verbindung VIII wird in einer Menge von 1 bis 10, vorzugsweise 1 bis 3, insbesondere 1 bis 1,5 Mol, bezogen auf 1 Mol der Verbindung VII, eingesetzt.

Die Reaktion wird in Gegenwart eines basischen Katalysators durchgeführt. Als solcher dient ein Alkalimetall, vorzugsweise Natrium, welches in einer Menge von 1 bis 30 Mol-%, vorzugsweise 2 bis 10 Mol-%, bezogen auf die Verbindung VII, verwendet wird.

Es ist jedoch auch möglich, die Verbindungen der Formel I auf direktem Wege aus den Verbindungen der Formel VII herzustellen, indem diese mit einer Verbindung der Formel IX umgesetzt werden:

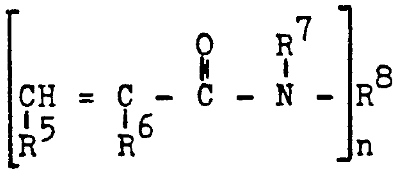

(IX).

n, $R^5$, $R^6$, $R^7$ und $R^8$ haben die oben angeführten Bedeutungen.

Die Reaktion wird wie oben angegeben bei einer Temperatur von 30 bis 150, vorzugeweise 50 bis 120, insbesondere 70 bis 120°C in Gegenwart eines Alkalimetalle, vorzugsweise Natrium, durchgeführt. Das Alkalimetall wird in einer Menge von 1 bis 30 Mol-%, vorzugsweise 2 bis 10 Mol-%, bezogen auf die Verbindung VII, verwendet. Die Verbindung IX wird in einer Menge von 1 bis 10, vorzugsweise 1 bis 3, insbesondere 1 bis 1,5 Mol, bezogen auf ein Mol der Verbindung VII, eingesetzt.

Bevorzugt ist die Umsetzung von Verbindungen der Formel V mit Verbindungen der Formel VI.

Geeignete Verbindungen der Formel VII sind beispielsweise
2-Butyl-7,7,9,9-tetramethyl-1-oxa-3,8-diaza-4-oxo-spiro-[4,5]-decan
2-iso-Butyl-7,7,9,9-tetramethyl-1-oxa-3,8-diaza-4-oxo-spiro-[4,5]-decan
2-Pentyl-7,7,9,9-tetramethyl-1-oxa-3,8-diaza-4-oxo-spiro-[4,5]-decan
2-iso-Pentyl-7,7,9,9-tetramethyl-1-oxa-3,8-diaza-4-oxo-spiro-[4,5]-decan
2-Hexyl-7,7,9,9-tetramethyl-1-oxa-3,8-diaza-4-oxo-spiro-[4,5]-decan
2-Heptyl-7,7,9,9-tetramethyl-1-oxa-3,8-diaza-4-oxo-spiro-[4,5]-decan
2-iso-Heptyl-7,7,9,9-tetramethyl-1-oxa-3,8-diaza-4-oxo-spiro-[4,5]-decan
2-Nonyl-7,7,9,9-tetramethyl-1-oxa-3,8-diaza-4-oxo-spiro-[4,5]-decan
2-iso-Nonyl-7,7,9,9-tetramethyl-1-oxa-3,8-diaza-4-oxo-spiro-[4,5]-decan
2-Undecyl-7,7,9,9-tetramethyl-1-oxa-3,8-diaza-4-oxo-spiro-[4,5]-decan
2-Phenyl-7,7,9,9-tetramethyl-1-oxa-3,8-diaza-4-oxo-spiro-[4,5]-decan
2-(4-Chlor-phenyl)-7,7,9,9-tetramethyl-1-oxa-3,8-diaza-4-oxo-spiro-[4,5]-decan
2-Ethyl-2,7,7,9,9-pentamethyl-1-oxa-3,8-diaza-4-oxo-spiro-[4,5]-decan
2-Propyl-2,7,7,9,9-pentamethyl-1-oxa-3,8-diaza-4-oxo-spiro-[4,5]-decan
2-iso-Propyl-2,7,7,9,9-pentamethyl-1-oxa-3,8-diaza-4-oxo-spiro-[4,5]-decan
2-Butyl-2,7,7,9,9-pentamethyl-1-oxa-3,8-diaza-4-oxo-spiro-[4,5]-decan
2-iso-Butyl-2,7,7,9,9-pentamethyl-1-oxa-3,8-diaza-4-oxo-spiro-[4,5]-decan
2-Pentyl-2,7,7,9,9-pentamethyl-1-oxa-3,8-diaza-4-oxo-spiro-[4,5]-decan
2-Hexyl-2,7,7,9,9-pentamethyl-1-oxa-3,8-diaza-4-oxo-spiro-[4,5]-decan
2-Nonyl-2,7,7,9,9-pentamethyl-1-oxa-3,8-diaza-4-oxo-spiro-[4,5]-decan
2,2,7,7,9,9-Hexamethyl-1-oxa-3,8-diaza-4-oxo-spiro-[4,5]-decan
2,2,7,7,8,9,9-Heptamethyl-1-oxa-3,8-diaza-4-oxo-spiro-[4,5]-decan
2,2-Diethyl-7,7,9,9-tetramethyl-1-oxa-3,8-diaza-4-oxo-spiro-[4,5]-decan
2,2-Dipropyl-7,7,9,9-tetramethyl-1-oxa-3,8-diaza-4-oxo-spiro-[4,5]-decan
2,2-Dibutyl-7,7,9,9-tetramethyl-1-oxa-3,8-diaza-4-oxo-spiro-[4,5]-decan
2-Ethyl-2-pentyl-7,7,9,9-tetramethyl-1-oxa-3,8-diaza-4-oxo-spiro-[4,5]-decan
2,2-Dibenzyl-7,7,9,9-tetramethyl-1-oxa-3,8-diaza-4-oxo-spiro-[4,5]-decan
2,2,4,4-Tetramethyl-7-oxa-3,13-diaza-14-oxo-dispiro-[5,1,4,2]-tetradecan
2,2,4,4-Tetramethyl-7-oxa-3,14-diaza-15-oxo-dispiro-[5,1,5,2]-pentadecan
2,2,4,4-Tetramethyl-7-oxa-3,20-diaza-21-oxo-dispiro-[5,1,11,2]-heneicosan
2,2,7,7,9,9-Hexamethyl-1-oxa-3,8-diaza-4-oxo-8-acetyl-spiro-[4,5]-decan
2,2,4,4-Tetramethyl-7-oxa-3,14-diaza-15-oxo-3-acetyl-dispiro-[5,1,5,2]-pentadecan
2,2,4,4-Tetramethyl-7-oxa-3,20-diaza-21-oxo-3-acetyl-dispiro-[5,1,11,2]-heneicosan

Beispiele für Verbindungen der Formel VI sind:
N-(2,2,6,6-Tetramethyl-4-piperidyl)-3-methoxipropylamin
N-(2,2,6,6-Tetramethyl-4-piperidyl)-3-ethoxipropylamin

N-(2,2,6,6-Tetramethyl-4-piperidyl)-3-octadecyloxipropylamin
N-(2,2,6,6-Tetramethyl-4-piperidyl)-3-dimethylaminopropylamin
N-(2,2,6,6-Tetramethyl-4-piperidyl)-2-dimethylaminoethylamin
N-(2,2,6,6-Tetramethyl-4-piperidyl)-4-diethylaminobutylamin
N-(2,2,6,6-Tetramethyl-4-piperidyl)-aminopropanol-3
N-(2,2,6,6-Tetramethyl-4-piperidyl)-4-diethylamino-1-methylbutylamin
2,2,6,6-Tetramethyl-4-aminopiperidin
N-(2,2,6,6-Tetramethyl-4-piperidyl)-butylamin
N-(2,2,6,6-Tetramethyl-4-piperidyl)-octadecylamin
N-(2,2,6,6-Tetramethyl-4-piperidyl)-cyclododecylamin
N-(2,2,6,6-Tetramethyl-4-piperidyl)-hexylamin
Di-(2,2,6,6-Tetramethyl-4-piperidyl)-amin,

Ammoniak, Butylamin, Dicyclohexylamin, Cyclododecylamin, Hexylamin, Dodecylamin, Octadecylamin, Dioctadecylamin, 2,3-Dimethylcyclohexylamin, 2,6-Dimethylmorpholin, Methylcyclohexylamin, Pyrrolidin, Piperidin, 3,5,5-Trimethylhexylamin, 2,2,-Dimethylpropylamin, Di-n-octylamin, Ethylendiamin, Propylendiamin, 1,6-Diaminohexan, 1,8-Diaminooctan, 1,12-Diaminododecan, 1,2-Diaminopropan, 2,5-Diamino-2,5-dimethylhexan, p-Phenylen-diamin, 1,8-Diamino-p-menthan,

1,3-Bis-(aminomethyl)-cyclohexan, 4,4'-Diaminodicyclohexylmethan, 3,3'-Dimethyl-4,4'-diaminodicyclohexylmethan, 4,4'-Diaminodiphenylmethan, 2(3),5(6)-Bis-(aminomethyl)-norbornan-Isomerengemisch, 3(4),8(9)-Bis-(aminomethyl)-tricyclo-[5.2.1.$0^{2,6}$]-decan-Isomerengemisch, Bis-(3-aminopropyl)-piperazin,

4,7-Dioxadecan-1,10-diamin, 4,9-Dioxadodecan-1,12-diamin, 7-Methyl-4,10-dioxatridecan-1,13-diamin; 3-Amino-1-methylaminopropan, 3-Amino-1-cyclohexyl-aminopropan,

3-Amino-1-(2-ethyl)-hexylaminopropan, N-(Cyclohexyl)-ethylendiamin, N-(Cyclohexyl)-1,6-diaminohexan, N-(2-Hydroxipropyl)-ethylendiamin,

N-(2,2,6,6-Tetramethyl-4-piperidyl)-ethylendiamin
N-(2,2,6,6-Tetramethyl-4-piperidyl)-1,6-diaminohexan
N-(2,2,6,6-Tetramethyl-4-piperidyl)-1,3-bis-(aminomethyl)-cyclohexan,
N-(2,2,6,6-Tetramethyl-4-piperidyl)-3(4),8(9)-bis-(aminomethyl)-tricyclo-[5.2.1.$0^{2,6}$]-decan-Isomerengemisch;
N,N'-Bis-(2,2,6,6-tetramethyl-4-piperidyl)-ethylendiamin
N,N'-Bis-(2,2,6,6-tetramethyl-4-piperidyl)-1,6-diaminohexan
N,N'-Bis-(2,2,6,6-tetramethyl-4-piperidyl)-3(4),8(9)-bis-(aminomethyl)-tricyclo-[5.2.1.$0^{2,6}$]-decan-Isomerengemisch
N,N'-Bis-(2,2,6,6-tetramethyl-4-piperidyl)-1,3-bis-(aminomethyl)-cyclohexan,
N,N'-Bis-(2,2,6,6-tetramethyl-4-piperidyl)-4,9-dioxadodecan-1,12-diamin,
N,N'-Bis-cyclododecyl-1,6-diaminohexan,
N,N'-Bis-isopropyl-1,6-diaminohexan,
N,N'-Bis-cyclohexyl-1,6-diaminohexan,
N,N'-Bis-(2,2,6,6-tetramethyl-4-piperidyl)-4,4'-diamino-dicyclohexylmethan,

N-(2,2,6,6-Tetramethyl-4-piperidyl)-N'-(cyclododecyl)-1,6-diaminohexan,
Geeignete Verbindungen der Formel VIII sind beispielsweise
Acrylsäuremethylester
Acrylsäureethylester
Acrylsäurebutylester
Methacrylsäuremethylester
Methacrylsäureethylester
Methacrylsäurebutylester
Crotonsäuremethylester
Crotonsäureethylester

Geeignete Verbindungen der Formel IX sind beispielsweise
Acrylsäureamid
Methacrylsäureamid
N,N'-Methylen-bis(acrylsäureamid)
N,N'-Ethylen-bis(acrylsäureamid)
N,N'-Hexamethylen-bis(acrylsäureamid)
Glyoxal-bis(acrylsäureamid)
Acrylsäure-2,2,6,6-tetramethylpiperid-4-yl-amid
Crotonsäure-2,2,6,6-tetramethylpiperid-4-yl-amid
Methacrylsäure-2,2,6,6-tetramethylpiperid-4-yl-amid
N,N'-Bis(2,2,6,6-tetramethylpiperid-4-yl)-N,N'-ethylen-bis(acrylsäureamid)
N,N'-Bis(2,2,6,6-tetramethylpiperid-4-yl)-N,N'-hexamethylen-bis(acrylsäureamid)

Die erfindungsgemäßen Verbindungen der Formel I zeigen gegenüber den Verbindungen der Deutschen Offenlegungsschrift 3 149 453 überraschend bessere Eigenschaften, die für ihren Einsatz als Lichtschutzmittel

für Polymere von entscheidender Bedeutung sind.

Für die Einarbeitung der Stabilisatoren in die Polymeren sowie für die Verarbeitung dieser stabilisierten Polymeren spielt die Flüchtigkeit und Thermostabilität eine wesentliche Rolle. Überraschenderweise zeigen die erfindungsgemäßen Verbindungen gegenüber den Verbindungen der Deutschen Offenlegungsschrift 3 149 453 wesentlich niedrigere Flüchtigkeiten.

Die erfindungsgemäßen Verbindungen der Formel (I) werden vor allem als Lichtstabilisatoren eingesetzt, beispielsweise für Polyolefine, insbesondere Polyethylen und Polypropylen, Ethylen-Propylen-Copolymere, Polybutylen, sowie Polystyrol, chloriertes Polyethylen, sowie Polyvinylchlorid, Polyester, Polycarbonat, Polymethylmethacrylate, Polyphenylenoxide, Polyamide, Polyurethane, Polypropylenoxid, Polyacetale, Phenol-Formaldehydharze, Epoxidharze, Polyacrylnitril und entsprechende Copolymerisate sowie ABS-Terpolymere. Vorzugsweise verwendet man die erfindungsgemäß hergestellten Verbindungen zum Stabilisieren von Polypropylen, niedermolekularen und hochmolekularen Polyethylen, Ethylen-Propylen-Copolymeren, Polyvinylchlorid, Polyester, Polyamid, Polyurethanen, Polyacrylnitril, ABS, Terpolymeren von Acrylester, Styrol und Acrylnitril, Copolymeren von Styrol und Acrylnitril oder Styrol und Butadien, insbesondere für Polypropylen, Polyethylen, Ethylen-Propylen-Copolymer oder ABS.

Die erfindungsgemäßen Verbindungen können auch für die Stabilisierung von Naturstoffen, beispielsweise Kautschuk, verwendet werden, sowie auch für Schmieröle. Weiterhin eignen sie sich auch für die Stabilisierung von Lacken.

Als Lacke kommen alle in der Industrielackierung verwendeten Arten, vorzugsweise Einbrennlacke, infrage. Letztere werden bei höherer Temperatur eingebrannt, um optimale Eigenschaften zu erhalten. Bevorzugt sind Naßlacke, welche als Bindemittel enthalten: Kombinationen aus ölmodifizierten Polyesterharzen (Ölalkydharze) und Melamin-Formaldehyd-Harzen oder Kombinationen aus fremdvernetzenden Polyacrylatharzen und Melamin-Formaldehyd-Harzen oder Kombinationen aus gesättigten Polyestern und Melamin-Formaldehyd-Harzen oder selbstvernetzende Polyacrylatharze oder auch Polyacrylatharze mit einpolymerisiertem Styrol. Weiterhin sind zu nennen Zweikomponenten-Acrylatharzlacke, bestehend aus hydroxylgruppenhaltigem Acrylatharz und aliphatischen oder aromatischen Isocyanaten, wie auch thermoplastische Polyacrylatharzlacke. Zu nennen sind auch Zweikomponenten-Polyurethanharzlacke, bestehend aus hydroxylgruppenhaltigen Polyesterharzen und/oder Polyätherharzen, gehärtet mit aliphatischen oder aromatischen Isocyanaten. Für Metallise-Lacke haben vor allem Bedeutung die thermoplastischen Polyacrylatharze oder fremdvernetzenden Polyacrylatharze in Kombination mit Butanol-verätherten Melaminharzen sowie auch hydroxygruppenhaltige Polyacrylatharze, gehärtet mit aliphatischen Isocyanaten. Ebenso kommen die an sich bekannten Pulverlacke infrage, welche z. B. mit einer Lösung der erfindungsgemäßen Verbindungen behandelt wurden.

Das Einbringen der erfindungsgemäßen Verbindungen in die zu schützenden Materialien erfolgt nach an sich bekannten Methoden, wobei auch Monomere oder Vorpolymerisate bzw. Vorkondensate mit diesen Stabilisatoren versehen werden können.

Neben den Verbindungen der Formel (1) können den Kunststoffen noch weitere Verbindungen zugesetzt werden. Derartige weitere Verbindungen sind z. B. Antioxidantien auf der Basis sterisch gehinderter Phenole, oder Schwefel oder Phosphor enthaltende Costabilisatoren oder eine Mischung von geeigneten sterisch gehinderten Phenolen und Schwefel und/oder Phosphor enthaltenden Verbindungen. Solche Verbindungen sind z. B. Benzofuranon(2)- und/oder Indolinon(2)Verbindungen, sterisch gehinderte Phenole wie β-(4-Hydroxy-3,5-di-t-butylphenyl)-propionsäurestearylester, Tetrakis-[methylen-3(3',5'-di-t-butyl-4-hydroxyphenyl-)propionat]-methan, 1,3,3-Tris-(2-methyl-4-hydroxy-5-t-butylphenyl)-butan, 1,3,5-Tris-(4-t-butyl-3-hydroxy-2,6-dimethylbenzyl)-1,3,5-triazin-2,4,6-(1H,3H,5H)-trion, Bis-(4-t-butyl-3-hydroxy-2,6-dimethylbenzyl)-dithiolterephthalat, Tris-(3,5-t-butyl-4-hydroxybenzyl)-isocyanurat, Triester der β-(4-Hydroxy-3,5-di-t-butylphenyl)propionsäure mit 1,3,4-Tris-(2-hydroxyethyl)-5-triazin-2,4,6-(1H,3H,5H)-trion, Bis-3,3-bis-(4'-hydroxy-3-t-butylphenyl)-butansäureglycolester, 2,3,5-Trimethyl-2,4,6-tris-(3,5-di-t-butyl-4-hydroxybenzyl-)benzol, 2,2'-Methylen-bis-(4-methyl-6-t-butylphenyl)-terephthalat, 4,4-Methylen-bis-(2,6-di-t-butylphenol), 4,4'-Butyliden-bis-(-t-butyl-meta-kresol), 4,4-Thio-bis-(2-t-butyl-5-methyl-phenol), 2,2'-Methylen-bis-(4-methyl-6-t-butyl-phenol). Auch antioxidativ wirkende Co-Stabilisatoren können zugegeben werden, wie z. B. schwefelhaltige Verbindungen, z. B. Distearylthiodipropionat, Dilaurylthiodipropionat, Tetrakis-(methylen-3-hexyl-thiopropionat)-methan, Tetrakis-(methylen-3-dodecylthiopropionat)-ethan und Dioctadecyldisulfid oder phosphorhaltige Verbindungen wie z. B. Trinonylphenylphosphit; 4,9-Distearyl-3,5,8,10-tetraoxadiphosphaspiroundecan, Tris-(2,4-t-butylphenyl)-phosphit oder Tetrakis-(2,4-di-t-butylphenyl)-4,4'-butylphenyl)-4,4'-biphenylen-diphosphonit.

Die Verbindungen der Formel I und deren oben erwähnte Mischungen kann man auch in Gegenwart weiterer Additive verwenden. Solche sind an sich bekannt und gehören z. B. zur Gruppe der Aminoacrylverbindungen, der UV-Absorber und Lichtschutzmittel wie die 2-(2'-Hydroxyphenyl)-benztriazole, 2-Hydroxybenzophenone, 1,3-Bis(2'-hydroxybenzoyl)-benzole, Salicylate, Zimtsäureester, Ester von gegebenenfalls substituierten Benzoesäuren, sterisch gehinderte Amine, Oxalsäurediamide.

Die Anwendungsmenge der erfindungsgemäßen Verbindungen der Formel I beträgt 0,01 - 5 Gew.-% bei Kunststoffen, 20 bis 80 Gew.-% bei Stabilisatorkonzentraten und 0,02 - 5 Gew.-% bei Lacken.

Die folgenden Beispiele dienen der Erläuterung der Erfindung.

**Beispiel 1**

Zu 150 g Xylol wurden 45,0 g (0,1 Mol) 2,2,4,4-Tetramethyl-7-oxa-3,20-diaza-21-oxo-dispiro-[5.1.11,2]-heneicos-20-yl-propansäuremethylester gegeben und am Wasserabscheider getrocknet. Dann gab man 0,4 g Tetraethylphosphoniumbromid 5,8 g (0,05 Mol) Hexamethylendiamin und 0,1 g Natriumhydrid zu. In 24 h wurden nahezu 3,2 g (0,1 Mol) Methanol über eine Kolonne abdestilliert. Nach dem Abkühlen wurde der Niederschlag abgesaugt. Durch Umkristallisieren aus Toluol erhielt man ein farbloses Produkt mit einem Schmelzpunkt von 179° C.

**Beispiel 2**

In 100 g Toluol wurden 36,4 g (0,1 Mol) 2,2,4,4-Tetramethyl-7-oxa-3,20-diaza-21-oxo-dispiro-[5.2.11,2]-heneicosan vorgelegt und am Wasserabscheider getrocknet. Dann wurden bei 80° C 0,4 g Triethylbenzylammoniumchlorid, 2,0 g Natrium und 11,2 g (0,05 Mol) N,N'-Methylen-bis(acrylsäureamid) zugegeben und der Ansatz 14 h bei 90° C gerührt. Der Niederschlag wurde bei Raumtemperatur abgesaugt und aus Toluol umkristallisiert. Man erhielt ein farbloses Produkt mit einem Schmelzpunkt von 148° C.

**Patentansprüche**

1. 1-Oxa-3,8-diaza-4-oxo-spiro-[4,5]-decan-Verbindungen der Formel I

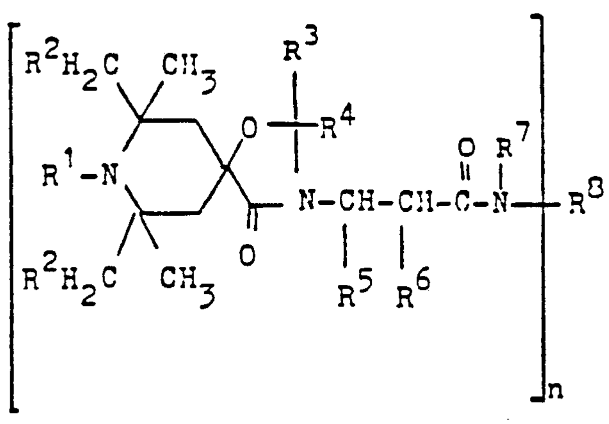

(I),

worin

| | |
|---|---|
| n | 1 oder 2 ist, |
| $R^1$ | Wasserstoff, $C_1$-$C_4$-Alkyl, Benzyl, Allyl, $C_2$-$C_{30}$-Alkanoyl, $C_3$-$C_{20}$-Alkenoyl, $C_7$-$C_{11}$-Aroyl, $C_8$-$C_{14}$-Arylalkanoyl oder $C_8$-$C_{20}$-Alkylaryl ist, |
| $R^2$ | Wasserstoff oder $C_1$-$C_4$-Alkyl bedeutet, |
| $R^3$ | Wasserstoff, $C_1$-$C_{18}$-Alkyl, $C_5$-$C_{12}$-Cycloalkyl, eine Phenyl- oder Naphthylgruppe, die durch Chlor oder $C_1$-$C_4$-Alkyl substituiert sein kann, oder eine gegebenenfalls durch $C_1$-$C_4$-Alkyl substituierte $C_7$-$C_{12}$-Phenylalkylgruppe ist, |
| $R^4$ | Wasserstoff, $C_1$-$C_4$-Alkyl, $C_5$-$C_{12}$-Cycloalkyl, durch -COOH, Carb-$C_1$-$C_4$-alkoxi oder Carbamoyl substituiertes $C_1$-$C_3$-Alkenyl, eine Phenyl-, Naphthyl- oder Pyridylgruppe, die durch $C_1$-$C_4$-Alkoxi oder $C_1$-$C_4$-Alkyl substituiert sein kann, oder eine $C_7$-$C_{12}$-Phenylalkylgruppe, die durch $C_1$-$C_4$-Alkyl substituiert sein kann, bedeutet, oder |
| $R^3$ und $R^4$ | zusammen mit dem sie bindendem Kohlenwasserstoff eine $C_5$-$C_{12}$-Cycloalkylengruppe, die durch eine bis vier $C_1$-$C_4$-Alkylgruppen substituiert sein kann, oder einen Rest der Formel II |

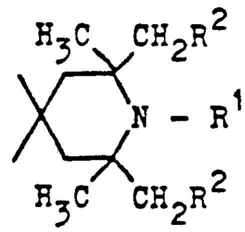

(II),

worin $R^1$ und $R^2$ die obengenannte Bedeutung haben, bilden,

$R^5$ Wasserstoff, Methyl, Phenyl oder Carb-$C_1$-$C_{21}$-alkoxi ist,

$R^6$ Wasserstoff oder Methyl bedeutet,

$R^7$ Wasserstoff, $C_1$-$C_{10}$-Alkyl oder ein Rest der Formel III

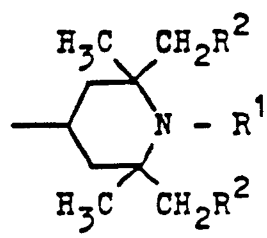

(III),

worin $R^1$ und $R^2$ die oben angegebene Bedeutung haben, ist,

$R^8$ bei n = 1

Wasserstoff, $C_1$-$C_{21}$-Alkyl, $C_2$-$C_{22}$-Alkenyl, $C_7$-$C_{18}$-Phenylalkyl, $C_5$-$C_{12}$-Cycloalkyl, Phenyl, Naphthyl, $C_7$-$C_{18}$-Alkylphenyl, $C_2$-$C_{20}$-Alkyl, welches unterbrochen sein kann durch -O- oder

-N-
|
$R^9$

und/oder substituiert sein kann durch einen Rest der Formel IV

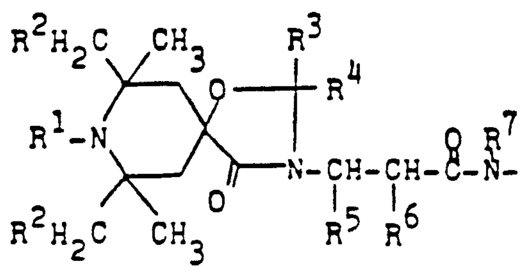

(IV),

oder durch $C_1$-$C_{21}$-Alkylcarboxyl, wobei $R^1$, $R^2$, $R^3$, $R^4$, $R^5$, $R^6$ und $R^7$ die obige Bedeutung haben und $R^9$ Wasserstoff, $C_1$-$C_{10}$-Alkyl oder ein Rest der Formel III ist, bedeutet,

$R^8$ bei n = 2

geradkettiges oder verzweigtes $C_1$-$C_{30}$-Alkylen, $C_6$-$C_{15}$-Cycloalkylen, $C_2$-$C_{30}$-Alkenylen, Phenyldialkylen, wobei diese Reste durch -O- oder

-N-,
|
$R^9$

worin $R^9$ die obige Bedeutung hat, unterbrochen sein können, bedeutet.

2. Verfahren zur Herstellung der Verbindungen der Formel I

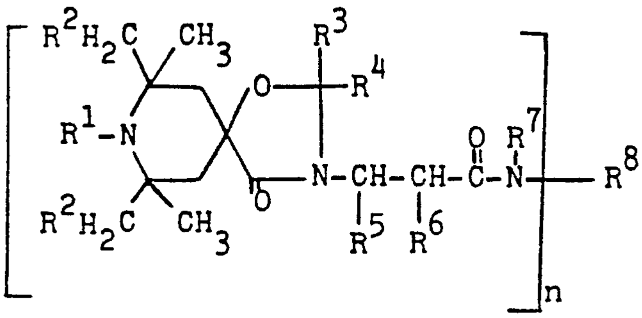

(I)

worin $R^1$, $R^2$, $R^3$, $R^4$, $R^5$, $R^6$, $R^7$, $R^8$ und n die in Anspruch 1 angeführten Bedeutungen haben, durch Umsetzung von 1-Oxa-3,8-diaza-4-oxo-spiro-[4,5]-decan-Verbindungen mit γ-δ-ungesättigten Verbindungen in einem inerten Lösemittel bei einer Temperatur von 30 bis 150°C in Gegenwart eines basischen Katalysators, dadurch gekennzeichnet, daß Verbindungen der Formel VII

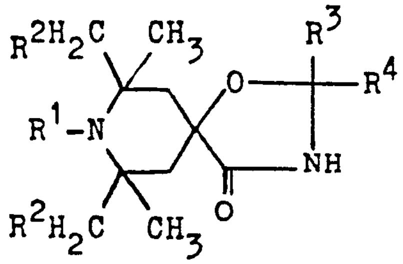

(VII),

worin $R^1$, $R^2$, $R^3$ und $R^4$ die oben angeführten Bedeutungen haben, entweder

a) zunächst mit Verbindungen der Formel VIII

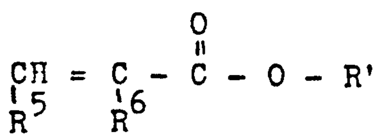

(VIII),

worin $R^5$ und $R^6$ die oben angeführten Bedeutungen haben und

R' $C_1$-$C_4$-Alkyl ist,

in Gegenwart von 0,05 bis 20 Mol-%, bezogen auf die Verbindung VII, eines Phasentransferkatalysators in einem bei Raumtemperatur flüssigen aromatischen Kohlenwasserstoff umgesetzt und danach die Reaktionsprodukte der Formel V

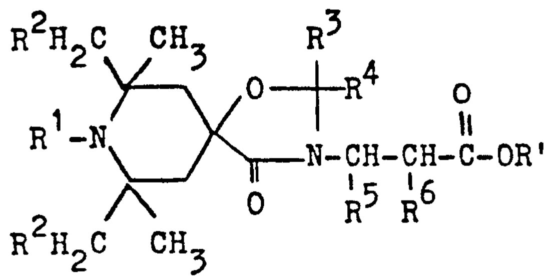

(V)

worin $R^1$, $R^2$, $R^3$, $R^4$, $R^5$, $R^6$ und R' die oben angeführten Bedeutungen haben, im gleichen Lösemittel bei der gleichen Temperatur mit Verbindungen der Formel VI

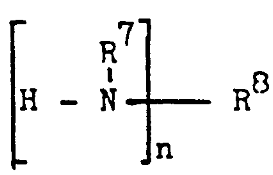

(VI)

worin n, $R^7$ und $R^8$ die in Anspruch 1 angeführten Bedeutungen haben, zur Reaktion gebracht werden, oder

b) mit Verbindungen der Formel IX

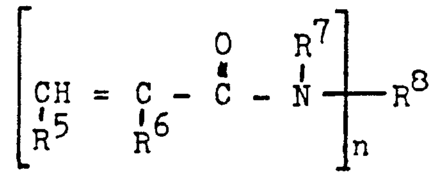

(IX),

worin n, $R^5$, $R^6$, $R^7$ und $R^8$ die oben angeführten Bedeutungen haben, in Gegenwart von 0,05 bis 20 Mol-%, bezogen auf die Verbindung VII, eines Phasentransferkatalysators in einem bei Raumtemperatur flüssigen aromatischen Kohlenwasserstoff umgesetzt werden.

3. Verfahren nach Anspruch 2, dadurch gekennzeichnet, daß man als Phasentransferkatalysator ein substituiertes Ammonium- oder Phosphoniumsalz oder einen Polyethylenglykoldialkylether verwendet.

4. Verfahren nach Anspruch 3, dadurch gekennzeichnet, daß man als Phasentransferkatalysator ein Tetraalkyl- oder Trialkylbenzylammoniumchlorid verwendet.

5. Verfahren nach Anspruch 3, dadurch gekennzeichnet, daß man als Phasentransferkatalysator ein Tetraalkylphosphoniumbromid verwendet.

6. Verwendung der Verbindungen nach Anspruch 1 zum Stabilisieren von synthetischen Polymeren.

7. Verwendung der Verbindungen nach Anspruch 1 zum Stabilisieren von Lacken.

8. Verfahren zum Stabilisieren von synthetischen Polymeren gegen den schädigenden Einfluß von Licht, dadurch gekennzeichnet, daß man den Polymeren, gegebenenfalls neben bisher bekannten, stabilisierend wirkenden Stoffen 0,01 bis 5 Gewichtsteile, bezogen auf Polymeres, eines Stabilisators nach Anspruch 1 zusetzt.

9. Verfahren zum Stabilisieren von Lacken gegen den schädigenden Einfluß von Licht, dadurch gekennzeichnet, daß man den Lacken, gegebenenfalls neben bisher bekannten, stabilisierend wirkenden Stoffen 0,02 bis 5 Gewichtsteile, bezogen auf Polymeres, eines Stabilisators nach Anspruch 1 zusetzt.

## Claims

1. A 1-oxa-3,8-diaza-4-oxo-spiro-[4,5]-decane compound of the formula I

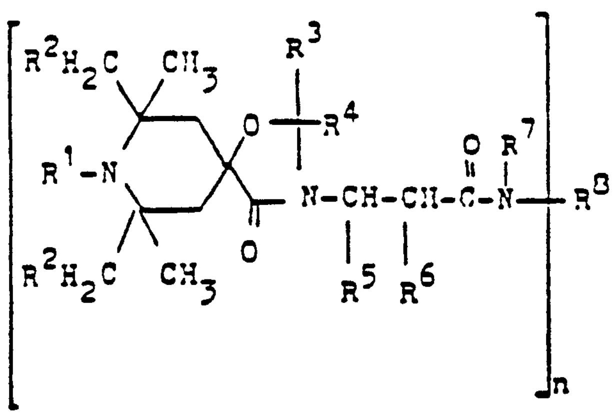

(I)

in which

| | |
|---|---|
| n | is 1 or 2, |
| $R^1$ | is hydrogen, $C_1$-$C_4$-alkyl, benzyl, allyl, $C_2$-$C_{30}$-alkanoyl, $C_3$-$C_{20}$-alkenoyl, $C_7$-$C_{11}$-aroyl, $C_8$-$C_{14}$-arylalkanoyl or $C_8$-$C_{20}$-alkylaryl, |
| $R^2$ | denotes hydrogen or $C_1$-$C_4$-alkyl, |
| $R^3$ | is hydrogen, $C_1$-$C_{18}$-alkyl, $C_5$-$C_{12}$-cycloalkyl, a phenyl or naphthyl group which can be substituted by chlorine or $C_1$-$C_4$-alkyl, or a $C_7$-$C_{12}$-phenylalkyl group which can be substituted by $C_1$-$C_4$-alkyl, |
| $R^4$ | denotes hydrogen, $C_1$-$C_4$-alkyl, $C_5$-$C_{12}$-cycloalkyl, $C_1$-$C_3$-alkenyl which is substituted by -COOH, carbo-$C_1$-$C_4$-alkoxy or carbamoyl, a phenyl, naphthyl or pyridyl group which can be substituted by $C_1$-$C_4$-alkoxy or $C_1$-$C_4$-alkyl, or a $C_7$-$C_{12}$-phenyl-alkyl group which can be substituted by $C_1$-$C_4$-alkyl or |
| $R^3$ and $R^4$ | together with the hydrocarbon linking them form a $C_5$-$C_{12}$-cycloalkylene group which can be substituted by one to four $C_1$-$C_4$-alkyl groups, or a radical of the formula II |

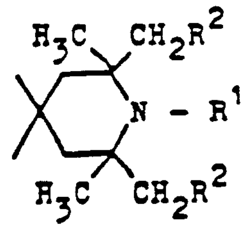

in which $R^1$ and $R^2$ have the abovementioned meaning,

$R^5$ is hydrogen, methyl, phenyl or carbo-$C_1$-$C_{21}$-alkoxy,

$R^6$ denotes hydrogen or methyl,

$R^7$ denotes hydrogen, $C_1$-$C_{10}$-alkyl or a radical of the formula III

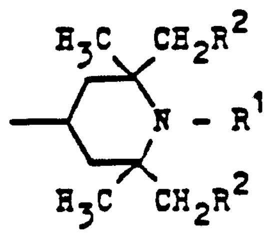

in which $R^1$ and $R^2$ have the abovementioned meaning,

$R^8$ in the case of n = 1 denotes hydrogen, $C_1$-$C_{21}$-alkyl, $C_2$-$C_{22}$-alkenyl, $C_7$-$C_{18}$-phenylalkyl, $C_5$-$C_{12}$-cycloalkyl, phenyl, naphthyl, $C_7$-$C_{18}$-alkylphenyl, $C_2$-$C_{20}$-alkyl which can be interrupted by -O- or

-N-
|
$R^9$

and/or can be substituted by a radical of the formula IV

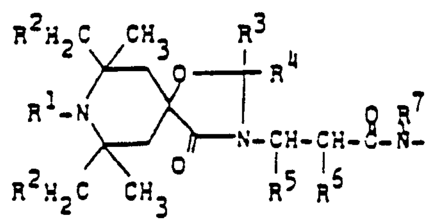

(IV)

or by $C_1$-$C_{21}$-alkylcarboxyl, $R^1$, $R^2$, $R^3$, $R^4$, $R^5$, $R^6$ and $R^7$ having the abovementioned meaning and $R^9$ being hydrogen, $C_1$-$C_{10}$-alkyl or a radical of the formula III,

$R^8$ in the case of n = 2 denotes straight-chain or branched $C_1$-$C_{30}$-alkylene, $C_6$-$C_{15}$-cycloalkylene, $C_2$-$C_{30}$-alkenylene or phenyldialkylene, it being possible for these radicals to be interrupted by -O- or

-N-,
|
$R^9$

in which $R^9$ has the abovementioned meaning.

2. A process for preparing a compound of the formula I

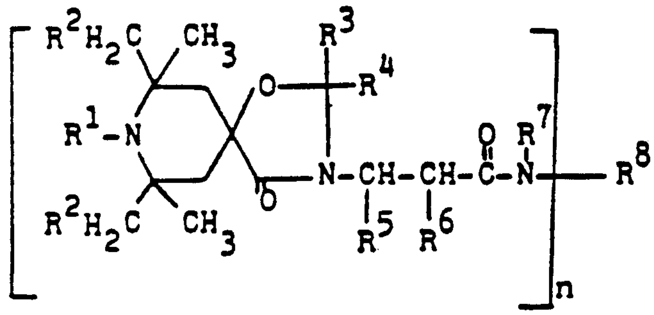

(I)

in which $R^1$, $R^2$, $R^3$, $R^4$, $R^5$, $R^6$, $R^7$, $R^8$ and n have the meanings mentioned in claim 1, by reacting 1-oxa-3,8-diaza-4-oxo-spiro-[4,5]-decane compounds with $\gamma$, -unsaturated compounds in an inert solvent at a temperature of 30 to 150° C in the presence of a basic catalyst, which comprises reacting compounds of the formula VII

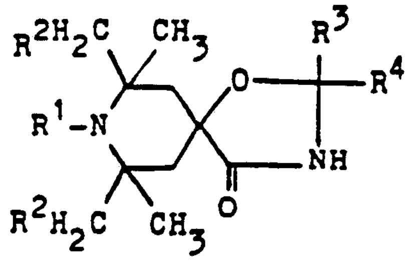

(VII)

in which $R^1$, $R^2$, $R^3$ and $R^4$ have the abovementioned meanings either

a) first with compounds of the formula VIII

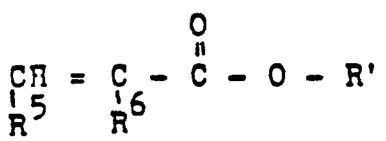

in which

$R^5$ and $R^6$ have the abovementioned meanings and

R' is $C_1$-$C_4$-alkyl,

in the presence of 0.05 to 20 mol-%, relative to compound VII, of a phase transfer catalyst in an aromatic hydrocarbon which is liquid at room temperature and then the reaction products of the formula V

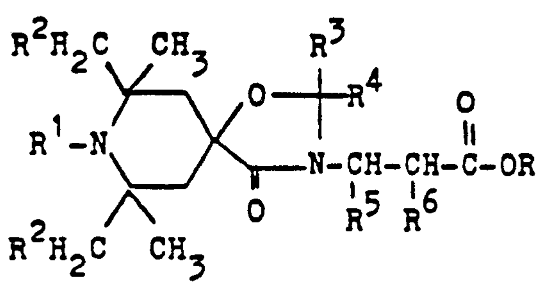

(V)

in which $R^1$, $R^2$, $R^3$, $R^4$, $R^5$, $R^6$ and R' have the abovementioned meanings, in the same solvent and at the same temperature with compounds of the formula VI

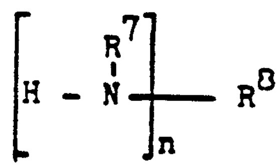

(VI)

in which n, $R^7$ and $R^8$ have the meanings mentioned in claim 1, or

b) with compounds of the formula IX

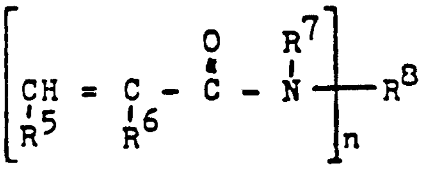

(IX)

in which n, $R^5$, $R^6$, $R^7$ and $R^8$ have the abovementioned meanings in the presence of 0.05 to 20 mol-%, relative to compound VII, of a phase transfer catalyst in an aromatic hydrocarbon which is liquid at room temperature.

3. The process as claimed in claim 2, wherein the phase transfer catalyst used is a substituted ammonium or phosphonium salt or a polyethylene glycol dialkyl ether.

4. The process as claimed in claim 3, wherein the phase transfer catalyst used is a tetraalkylammonium or trialkylbenzyl-ammonium chloride.

5. The process as claimed in claim 3, wherein the phase transfer catalyst used is a tetraalkylphosphonium bromide.

6. Use of the compound as claimed in claim 1, for stabilizing synthetic polymers.

7. Use of the compound as claimed in claim 1, for stabilizing surface coatings.

8. A process for stabilizing synthetic polymers against the damaging effect of light, which comprises adding to the polymers, if desired in addition to hitherto disclosed stabilizing substances, 0.01 to 5 parts by weight, relative to polymer, of the stabilizer as claimed in claim 1.

9. A process for stabilizing surface coatings against the damaging effect of light, which comprises adding to the surface coatings, if desired in addition to hitherto disclosed stabilizing substances, 0.02 to 5 parts by weight, relative to polymer, of the stabilizer as claimed in claim 1.

**Revendications**

1. Oxa-1 diaza-3,8 oxo-4 spiro-[4.5]-décanes répondant à la formule:

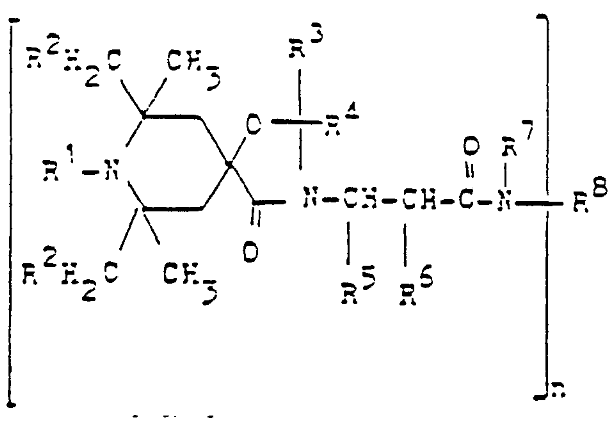

(I)

dans laquelle:

n est égal à 1 ou à 2,

$R^1$ représente l'hydrogène, un alkyle en $C_1$-$C_4$, un benzyle, un allyle, un alcanoyle en $C_2$-$C_{30}$, un alcénoyle en $C_3$-$C_{20}$, un aroyle en $C_7$-$C_{11}$, un aryl-alcanoyle en $C_8$-$C_{14}$ ou un alkylaryle en $C_8$-$C_{20}$,

$R^2$ représente l'hydrogène ou un alkyle en $C_1$-$C_4$,

$R^3$ représente l'hydrogène, un alkyle en $C_1$-$C_{18}$, un cycloalkyle en $C_5$-$C_{12}$, un radical phényle ou naphtyle éventuellement porteur d'un atome de chlore ou d'un alkyle en $C_1$-$C_4$, ou un radical phénylalkyle en $C_7$-$C_{12}$ éventuellement porteur d'un alkyle en $C_1$-$C_4$,

$R^4$ représente l'hydrogène, un alkyle en $C_1$-$C_4$, un cycloalkyle en $C_5$-$C_{12}$, un alcényle en $C_1$-$C_3$ porteur d'un -COOH, d'un alcoxycarbonyle à alcoxy en $C_1$-$C_4$ ou d'un carbamoyle, un radical phényle, naphtyle ou pyridyle éventuellement porteur d'un alcoxy en $C_1$-C ou d'un alkyle en $C_1$-$C_4$, ou un radical phénylalkyle en $C_7$-$C_{12}$ éventuellement porteur d'un alkyle en $C_1$-$C_4$, ou

$R^3$ et $R^4$ forment ensemble, et avec l'atome de carbone qui les unit, un radical cycloalkylène en $C_5$-$C_{12}$ éventuellement porteur d'un à quatre alkyles en $C_1$-$C_4$, ou un radical de formule II:

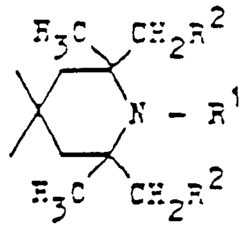

(II)

dans lequel $R^1$ et $R^2$ ont les significations précédemment données,

$R^5$ représente l'hydrogène, un méthyle, un phényle ou un alcoxycarbonyle à alcoxy en $C_1$-$C_{21}$,

$R^6$ représente l'hydrogène ou un méthyle,

$R^7$ représente l'hydrogène, un alkyle en $C_1$-$C_{10}$ ou un radical de formule III:

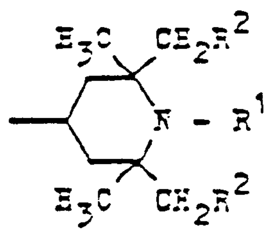

(III)

dans lequel $R^1$ et $R^2$ ont les significations précédemment données,

$R^8$ représente, dans le cas où n est égal à 1:
l'hydrogène, un alkyle en $C_1$-$C_{21}$ un alcényle en $C_2$-$C_{22}$, un phénylalkyle en $C_7$-$C_{18}$, un cycloalkyle en $C_5$-$C_{12}$, un phényle, un naphtyle, un alkylphényle en $C_7$-$C_{18}$, ou un alkyle en $C_2$-$C_{20}$ éventuellement interrompu par -O- ou par

-N-<br>|<br>$R^9$

et/ou éventuellement porteur d'un radical de formule IV:

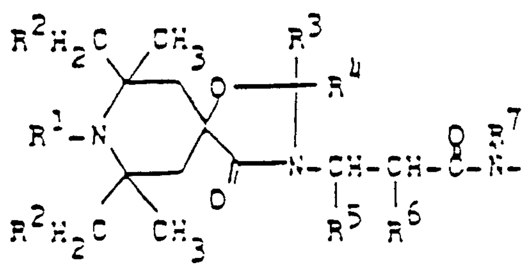

(IV)

ou d'un alkylcarbonyle en $C_1$-$C_{21}$, les symboles $R^1$, $R^2$, $R^3$, $R^4$, $R^5$, $R^6$ et $R^7$ ayant les significations précédemment données et $R^9$ représentant l'hydrogène, un alkyle en $C_1$-$C_{10}$ ou un radical de formule III, et

$R^8$ représente, dans le cas où n est égal à 2:
un alkylène en $C_1$-$C_{30}$ linéaire ou ramifié, un cycloalkylène en $C_6$-$C_{15}$, un alcénylène en $C_2$-$C_{30}$ ou un phényl-dialkylène, ces radicaux pouvant être interrompus par -O- ou par

-N-, .<br>|<br>$R^9$

le symbole $R^9$ ayant la signification précédemment donnée.

2. Procédé pour préparer des composés répondant à la formule I:

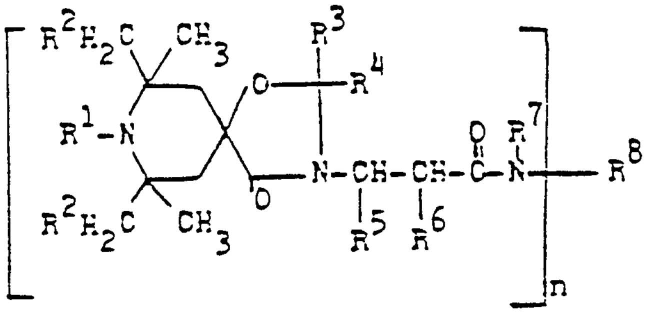 (I)

dans laquelle $R^1$, $R^2$, $R^3$, $R^4$, $R^5$, $R^6$, $R^7$, $R^8$ et n ont les significations qui leur ont été données à la revendication 1, par réaction d'oxa-1 diaza-3,8 oxo-4 spiro-[4.5]-décanes avec des composés insaturés en γ, δ, dans un solvant inerte, à une température de 30 à 150°C en présence d'un catalyseur basique, procédé caractérisé en ce qu'on fait réagir des composés répondant à la formule VII:

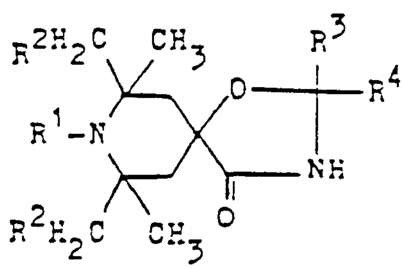 (VII)

dans laquelle $R^1$, $R^2$, $R^3$, et $R^4$ ont les significations précédemment données, soit:

a) d'abord avec des composés répondant à la formule VIII:

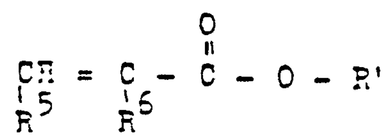 (VIII),

dans laquelle

$R^5$ et $R^6$ ont les significations précédemment données et

R' représente un alkyle en $C_1$-$C_4$,

en présence de 0,05 à 20 % en moles, par rapport au composé VII, d'un catalyseur de transfert de phase, dans un hydrocarbure aromatique liquide à la température ambiante, et on fait ensuite réagir les produits réactionnels répondant à la formule V:

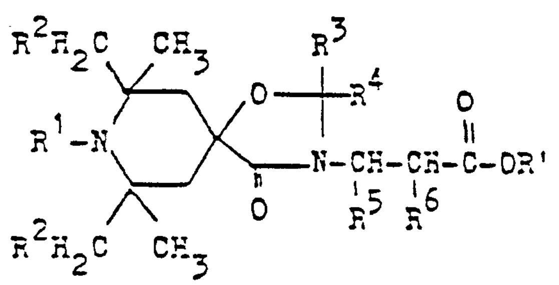 (V)

dans laquelle $R^1$, $R^2$, $R^3$, $R^4$, $R^5$, $R^6$ et R' ont les significations précédemment données, dans le même solvant et à la même température, avec des composés répondant à la formule VI:

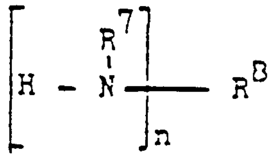 (VI)

dans laquelle n, $R^7$ et $R^8$ ont les significations données à la revendication 1,

soit :

b) avec des composés répondant à la formule IX :

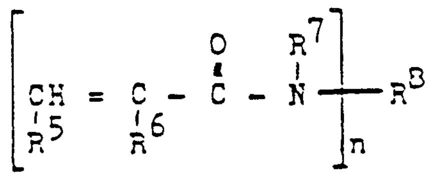 (IX)

dans laquelle n, $R^5$, $R^6$, $R^7$ et $R^8$ ont les significations précédemment données, en présence de 0,05 à 20 % en moles, par rapport au composé VII, d'un catalyseur de transfert de phase, dans un hydrocarbure aromatique liquide à la température ambiante.

3. Procédé selon la revendication 2 caractérisé en ce qu'on utilise, comme catalyseur de transfert de phase, un sel d'ammonium ou de phosphonium substitué ou un éther dialkylique de polyéthylène-glycol.

4. Procédé selon la revendication 3 caractérisé en ce qu'on utilise, comme catalyseur de transfert de phase, un chlorure de tétraalkyl-ammonium ou de trialkylbenzyl-ammonium.

5. Procédé selon la revendication 3 caractérisé en ce qu'on utilise, comme catalyseur de transfert de phase, un bromure de tétraalkyl-phosphonium.

6. Application des composés selon la revendication 1 à la stabilisation de polymères synthétiques.

7. Application des composés selon la revendication 1 à la stabilisation de peintures et de vernis.

8. Procédé pour stabiliser des polymères synthétiques contre l'action destructrice de la lumière, procédé caractérisé en ce qu'on ajoute aux polymères, éventuellement en plus de composés stabilisants connus, de 0,01 à 5 parties en poids, par rapport au polymère, d'un stabilisant selon la revendication 1.

9. Procédé pour stabiliser des peintures et des vernis contre l'action destructrice de la lumière, procédé caractérisé en ce qu'on ajoute aux peintures ou aux vernis, éventuellement en plus de composés stabilisants connus, de 0,02 à 5 parties en poids, par rapport au polymère, d'un stabilisant selon la revendication 1.